(19) Europäisches Patentamt / European Patent Office / Office européen des brevets

(11) **EP 3 985 086 B1**

(12) **EUROPEAN PATENT SPECIFICATION**

(45) Date of publication and mention
of the grant of the patent:
**28.05.2025  Bulletin 2025/22**

(21) Application number: **21203496.1**

(22) Date of filing: **19.10.2021**

(51) International Patent Classification (IPC):
**C09K 21/08** (2006.01)     **A62D 1/00** (2006.01)

(52) Cooperative Patent Classification (CPC):
**A62D 1/0092; C09K 21/08**

(54) **FIRE SUPPRESSION COMPOSITIONS**

FEUERLÖSCHZUSAMMENSETZUNGEN

COMPOSITIONS DE LUTTE CONTRE LES INCENDIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.10.2020  US 202017073515**

(43) Date of publication of application:
**20.04.2022  Bulletin 2022/16**

(73) Proprietor: **Kidde Technologies, Inc.
Wilson, NC 27896 (US)**

(72) Inventors:
• **Simpson, Terry**
  **Wake Forest (US)**
• **Liu, Qing**
  **Wake Forest (US)**
• **Papas, Paul**
  **West Hartford (US)**
• **Soteriou, Marios C**
  **Middletown (US)**
• **Fazzio, Mark P**
  **Wilson (US)**
• **Baldwin, Eli**
  **Knightdale (US)**
• **Chattaway, Adam**
  **Windsor (GB)**

(74) Representative: **Dehns
10 Old Bailey
London EC4M 7NG (GB)**

(56) References cited:
WO-A1-2019/099961     WO-A2-2021/230935
CN-A- 106 281 232     KR-A- 20010 038 267
US-A1- 2015 041 157

**Description**

TECHNICAL FIELD

The present disclosure relates to fire suppression compositions comprising $CF_3I$ and $CO_2$.

BACKGROUND

Halon 1301 (bromotrifluoromethane, $CF_3Br$) has frequently been employed as a fire suppression agent. However, production of this agent was banned in 1994 due to its high ozone depleting potential. There is therefore a desire to replace Halon 1301 with more environmentally friendly fire suppression agents. A promising alternative to Halon 1301, $CF_3I$ (trifluoroiodomethane), failed a key fire test and can be subject to decomposition during use. A solution must be found that will improve the stability of the alternative fire suppression agents. US 2015/0041157 discloses fire suppressing mixtures containing an organic suppressant, a halogen element and an organic compound. KR 2001 0038267 discloses fire extinguishing compositions containing trifluoroiodomethane and carbon dioxide. CN 106 281 232 and WO 2019/099961 disclose refrigerant compositions.

SUMMARY

This disclosure relates to fire suppression compositions comprising blends of $CF_3I$ and $CO_2$ as set out in claim 1.
The $CF_3I$ is present in an amount from 53 to 59 mol.%, e.g. 54 mol.% to 59 mol.%, 55 mol.% to 59 mol.%, 56 to 59 mol.%, or 58 to 59 mol.%, based on the total moles of $CF_3I$ and $CO_2$ present in the fire suppression composition.
The amount of $CF_3I$, expressed as a percentage of the total amount of $CF_3I$ and $CO_2$ in the fire suppression compositions, may also be expressed in weight %.
In a further aspect of the present disclosure, the $CF_3I$ is present in an amount of 83 weight% to 86 weight%, based on the total weight of $CF_3I$ and $CO_2$ present in the fire suppression composition.
The $CF_3I$ may be present in an amount of from 84 weight% to 86 weight%, or 85 weight% to 86 weight%, based on the total weight of $CF_3I$ and $CO_2$ present in the fire suppression composition.
As the amount of $CF_3I$ is expressed as proportion of the total of $CF_3I$ and $CO_2$, it will be understood that the percentage of $CO_2$ (also expressed as a proportion of the total) is the remainder of this total (i.e. 100 minus the percentage of $CF_3I$). Where the composition consists of, or consists essentially of, $CF_3I$ and $CO_2$, these proportions will also be applicable to the composition as a whole. Fire suppression compositions comprising such blends of $CF_3I$ and $CO_2$, e.g. those with molar ratios of $CF_3I$ to $CO_2$ of from 3:7 to 7:13, or 2:1 to 5:1 form a further aspect of this disclosure.
In addition to $CF_3I$ and $CO_2$, the fire suppression compositions as disclosed herein can further comprise one or more additional components. The additional components may be selected from a gas (e.g. an inert gas), an additional fire suppressant compound, odorants, or combinations thereof.
The total amount of additional components, if present, is up to 18 weight%, based on the total weight of the fire suppression composition. The $CF_3I$ and $CO_2$ (in total) is present in an amount of at least 82 weight%, based on the total weight of the fire suppression composition.
The total amount of additional components present in the fire suppression composition may be up to 15 weight%, up to 10 weight%, up to 8 weight%, up to 5 weight% or up to 3 weight%, (e.g. from 0.1 weight% up to these limits) based on the total weight of the fire suppression composition. The remainder is $CF_3I$ and $CO_2$, based on the total weight of the fire suppression composition, e.g. at least 82, at least 85, at least 90, at least 92, at least 95 or at least 97 weight% of the fire suppression composition is the $CF_3I/CO_2$ blend. In some aspects, the total amount of additional components present in the fire suppression composition is up to 2 weight% or up to 1 weight%, e.g. 0.1 weight% to 2 weight % or 0.1 weight % to 1 weight % (e.g. at least 98, at least 99, e.g. 98-99.9 or 99-99.9 weight% of the fire suppression composition is the $CF_3I/CO_2$ blend).
The additional components, if present, may be one or more gases, e.g. an inert gas, or a propellant. Examples of suitable gases include nitrogen, argon, helium and neon, and combinations thereof.
The optional gas may be present in an amount of up to 1 weight%, based on the total weight of the fire suppression composition. For example, the gas may be present in an amount of up to 0.9 weight%, up to 0.8 weight%, up to 0.7 weight%, up to 0.6 weight%, up to 0.5 weight%, up to 0.4 weight%, up to 0.3 weight%, up to 0.2 weight% or up to 0.1 weight%, based on the total weight of the fire suppression composition. If present, a lower limit for the gas may be 0.05 weight %.
The additional component, if present, may be an additional fire suppressant compound, i.e. one that is not $CF_3I$ or $CO_2$.
The additional fire suppressant compound, if present, may be present in an amount of up to 18 weight%, based on the total weight of the fire suppression composition. For example, the total amount of additional fire suppressant compound present in the fire suppression composition may be up to 15 weight%, up to 10 weight%, up to 8 weight%, up to 5 weight% or up to 3 weight% based on the total weight of the fire suppression composition. If present, a lower limit for the additional suppressant may be 0.1 weight %.

The additional component, if present, can be an odorant. Examples of odorants include compounds which include one or more carbon-carbon double bonds, and/or compounds which are aromatic. The odorant compounds may further include a hydroxyl group, an iodine group, or both.

The odorant compound, if present, may be present in an amount of up to 1 weight% based on the total weight of the fire suppression composition. For example, the odorant may be present in an amount of up to 0.9 weight%, up to 0.8 weight%, up to 0.7 weight%, up to 0.6 weight%, up to 0.5 weight%, up to 0.4 weight%, up to 0.3 weight%, up to 0.2 weight% or up to 0.1 weight%, based on the total weight of the fire suppression composition. If present, a lower limit for the odorant may be 0.05 weight %.

The present disclosure also provides a device, e.g. a fire extinguisher, fire suppression device, or storage device, comprising a fire suppression composition as herein described.

Also disclosed is a device, e.g. a fire extinguisher, fire suppression device, or storage device, comprising at least two separate containers, wherein the first container comprises $CF_3I$ and the second container comprises $CO_2$. The proportions of the $CF_3I$ and the $CO_2$ are as described herein. The contents of the containers can be combined immediately prior to use to produce a fire suppression composition as herein described. As would be understood, the first and/or second container can comprise one or more additional components (e.g. one or more additional components as herein described) or any additional components can be stored in a further container or containers.

Disclosed is a fire suppression system or device comprising a fire suppression composition as herein described, or the components thereof. The fire suppression system can comprise a fire suppression composition herein described and a dispensing component (such as one or more nozzles that disperse the fire suppression composition). In an alternative aspect, the fire suppression system can contain: (i) two separate containers, wherein the first container comprises $CF_3I$ and the second container comprises $CO_2$, and (ii) a combining and dispensing component which is configured to combine the contents of the separate containers to form a fire suppression composition as herein described, and then dispense said resulting fire suppression composition.

Also disclosed is a method for extinguishing a fire comprising using a fire suppression composition as herein described.

Disclosed is a method for preparing a fire suppression composition as herein described, said method comprising combining $CF_3I$ and $CO_2$ such that $CF_3I$ is present in an amount as herein described in relation to the total amount of $CF_3I$ and $CO_2$. The method may comprise the steps of (i) providing $CF_3I$, (ii) providing $CO_2$ and (iii) combining $CF_3I$ and $CO_2$ such that $CF_3I$ is present in an amount as herein described in relation to the total amount of $CF_3I$ and $CO_2$. The method can further comprise the additional step of adding one or more additional components as herein described.

In some aspects, the fire suppression composition of the present disclosure consists of, or consists essentially of, $CF_3I$ and $CO_2$ in the proportions described herein.

DETAILED DESCRIPTION

$CF_3I$ is an environmentally friendly alternative to fire suppression agents like Halon 1301 because $CF_3I$ has a lower ozone depletion potential. The lower ozone depletion potential is due to the lower stability of the molecule. However, the lower stability (or the increased tendency to degrade) presents a challenge for use of $CF_3I$ or blends containing $CF_3I$ as a fire suppression agent. The lower stability has discouraged the use of $CF_3I$ in fire suppression applications as it can decompose, thus reducing its efficacy. The present disclosure involves addition of $CO_2$ to the $CF_3I$, which has been found to improve stability of $CF_3I$.

When released, the $CO_2$ is able to remove a large amount of heat from its surroundings (i.e. has a high heat capacity). This temperature reduction can reduce the severity of the fire, as well as reducing the decomposition rate of $CF_3I$, maximizing the available $CF_3I$ present when the fire suppression composition is used to extinguish a fire.

The presence of $CO_2$ in the fire suppression composition can reduce the temperature of the atmosphere in the space to be protected to below 370 °C (700°F), e.g. to below 360°C, to below 350°C, to below 340°C, to below 330°C, to below 320°C, or to below 315°C (600 °F).

$CO_2$ is a physically acting fire suppression agent and $CF_3I$ is a chemically acting agent. Combining these two different types of agent as described herein results in a synergistic combination. More specifically, the blends of $CO_2$ and $CF_3I$ as disclosed herein have been shown to be a synergistic combination. The combination of these two components has surprisingly resulted in a fractional inerting composition number of less than the sum of the two components when measured separately. The effect of this is that the combination of these two components has an enhanced ability to extinguish a fire than the two components would have had if used separately in the same amount.

It has also been found that fire suppression compositions according to the present disclosure can have a vapor pressure in a similar range as that of conventional fire suppression agents such as Halon 1301. The vapor pressure allows the fire suppression composition to be used in conventional hardware such as preexisting fire extinguishing containers and devices.

The present disclosure will now be further described by way of the following non-limiting examples.

EXAMPLES

EXAMPLE 1 - INERTING TESTS

Testing Procedure

Testing was carried out against propane-air explosions in a 42 L sphere. The most explosive propane-air mixture is 4% propane in air. This concentration was therefore used to assess the relative performance of extinguishing agents and blends thereof.

The sphere was evacuated. Whilst monitoring the pressure transducer, propane was added to a pressure of 0.04 atm (4% in the final mix). The agent or agents were added at the desired concentration. Air was then added to raise the pressure in the sphere to 1.00 atm. A fan can then be used to ensure that all the gases are mixed homogeneously throughout the sphere. A spark was ignited using a center point spark ignition and the pressure rise was monitored by a data logger. A pressure rise of 1psi or lower is designated as a pass. The standards used for inerting testing are:

ASTM E2079-07 - the standard test method for limiting oxidant concentration in gases and vapors
BS EN 1839:2012 - determination of explosion limits in gases and vapors
BS EN 15967:2012 - determination of maximum explosion pressure and the maximum rate of pressure rise of gases and vapors.

Fractional Inerting Contribution

When assessing blends of components, the concept of fractional inerting contribution is used. This is defined as:

$$FIC = \sum_{i=1}^{n} \frac{C_i}{IC_i}$$

Where $C_i$ is the concentration of component i
And $IC_i$ is the inerting concentration of component i.

Thus, inerting should be attained when FIC = 1 (i.e. the sum of individual concentrations has reached the overall required amount to achieve inerting). It therefore follows that if inerting is achieved at FIC less than 1, then the blend is more effective than the sum of its components. In other words, the blend is exhibiting synergy.

Blends of $CF_3I$ and $CO_2$

Blends of $CF_3I$ and $CO_2$ were evaluated and it was found that successful inerting results were found at FIC values of lower than 1:

In Table I and II, values expressed in psig are to be converted in KPa, with 1 psig=6.89476 kPa.

Table I

| Example No. | Propane (Vol%) | CF₃I (Vol%) | CO₂ (Vol%) | Mol Ratio | Pres rise (psig) | FIC | Rel. wt to 6% Halon 1301 | Rel. vol to 6% Halon 1301 | Mol. % CF₃I* |
|---|---|---|---|---|---|---|---|---|---|
| 1.1 | 4.00 | 2.32 | 11.51 | 1:5 | **0.73** | 0.77 | 1.07 | 1.58 | 16.8 |
| 1.2 | 3.97 | 2.63 | 10.36 | 1:4 | **0.36** | 0.77 | 1.07 | 1.49 | 20.25 |
| 1.3 | 3.98 | 2.93 | 8.64 | 1:3 | **0.99** | 0.76 | 1.04 | 1.36 | 25.3 |
| 1.4 | 4.01 | 3.24 | 7.42 | 3:7 | **0.91** | 0.76 | 1.04 | 1.27 | 30.4 |
| 1.5 | 4.02 | 3.31 | 6.92 | 6:13 | **0.78** | 0.76 | 1.03 | 1.22 | 32.4 |

(continued)

| Example No. | Propane (Vol%) | CF$_3$I (Vol%) | CO$_2$ (Vol%) | Mol Ratio | Pres rise (psig) | FIC | Rel. wt to 6% Halon 1301 | Rel. vol to 6% Halon 1301 | Mol. % CF$_3$I* |
|---|---|---|---|---|---|---|---|---|---|
| 1.6 | 4.05 | 3.49 | 6.46 | 7:13 | **0.9** | 0.77 | 1.04 | 1.2 | 35.1 |
| *Mol.% CF$_3$I expressed as a proportion of the moles of CF$_3$I and CO$_2$ | | | | | | | | | |

Table II

| Example No. | Propane (Vol%) | CF$_3$I (Vol%) | CO$_2$ (Vol%) | Mol Ratio | Pres rise (psig) | FIC | Rel. wt to 6% Halon 1301 | Rel. vol to 6% Halon 1301 | Mol. % CF$_3$I* |
|---|---|---|---|---|---|---|---|---|---|
| 1.7 | 4.02 | 4.05 | 3.33 | 6:5 | **0.33** | 0.74 | 1.01 | 0.96 | 54.9 |
| 1.8 | 4.01 | 4.15 | 3.31 | 5:4 | **0.64** | 0.76 | 1.03 | 0.97 | 55.6 |
| 1.9 | 4.00 | 4.26 | 2.92 | 3:2 | **0.67** | 0.76 | 1.03 | 0.95 | 59.3 |
| 1.10 | 4.04 | 5.19 | 2.53 | 2:1 | **0.94** | 0.89 | 1.22 | 1.06 | 67.2 |
| 1.11 | 3.98 | 5.47 | 1.76 | 3:1 | **0.97** | 0.90 | 1.25 | 1.03 | 75.7 |
| 1.12 | 4.08 | 6.01 | 1.49 | 4:1 | **0.97** | 0.98 | 1.36 | 1.09 | 80.1 |
| 1.13 | 4.01 | 6.49 | 1.3 | 5:1 | **0.96** | 1.04 | 1.46 | 1.15 | 83.3 |
| *Mol.% CF$_3$I expressed as a proportion of the moles of CF$_3$I and CO$_2$ | | | | | | | | | |

Examples 1.1 to 1.6 and 1.10 to 1.13 are outside the scope of the present claims.

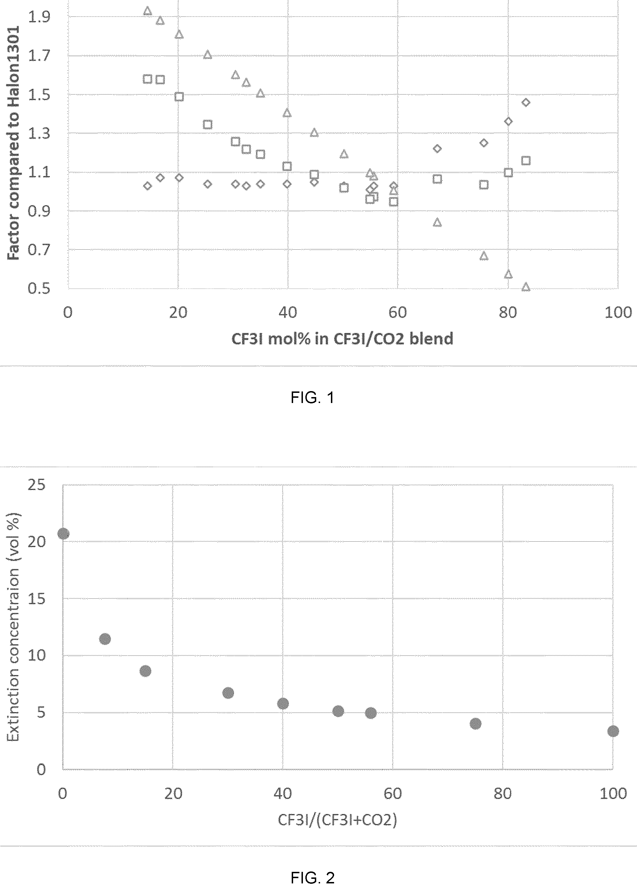

Figure 1 shows the results of these inerting tests on CF$_3$/CO$_2$ blends, in which:

Diamonds represent the relative weight of the blend to 6% Halon 1301

Squares represent the relative volume of the blend to 6% Halon 1301

Triangles represent the relative pressure of the blend to 6% Halon 1301

For all three parameters, a lower value relative to Halon 1301 is desired. Examining Figure 1 in detail:

• The relative weight appears approximately constant over the range 15 to 60% CF$_3$I, then it increases.

• Relative volume decreases from 15 to 60% then increases.

• Relative pressure decreases constantly from 15 to 85% CF$_3$I.

• Thus there is an optimum region (ca. 55%-60 mol% CF$_3$I) where all three parameters are low.

These results show the equivalent weight of CF$_3$I/CO$_2$ blend to Halon 1301 to achieve same inerting efficiency. The 56/44 by mol.% CF$_3$I/CO$_2$ blend showed inerting equivalent to Halon 1301 at only 1.03x weight, 0.97x volume and 1.08 x the relative pressure of a Halon 1301 system.

As can be seen from Tables I and II above and Figure 1, blends according to the present disclosure show a synergistic effect between the CF$_3$I and CO$_2$ in the blend. Examples 1.3-1.6 show a particularly good synergistic effect, coupled with an acceptable vapor pressure/temperature characteristics. Examples 1.7-1.12 show a synergistic effect, and these examples have improved vapor pressure/temperature characteristics.

EXAMPLE 2 - CUP BURNER TESTING

A cup burner is a relatively simple apparatus used to measure the extinguishing concentration of a fire extinguishing agent, or in this case, a blend of two agents. The cup burner test is quick to perform, uses little extinguishing agent and gives repeatable results. It is regarded as an industry standard test for evaluating fire extinguishing agents. This test was carried out to investigate the equivalent weight of $CF_3I/CO_2$ blend to Halon 1301 to achieve same efficiency.

A flame was established in a cup, situated in the centre of a glass tube. There was an airflow in the tube to feed the flame. Into this airflow the extinguishing agent was introduced, and its concentration was gradually increased until the flame was extinguished. The agent concentration was measured, and the test was then repeated.

The results are shown in Table III below:

Table III

| $CF_3I$ proportion (%) | 0 | 7.5 | 15 | 30 | 40 | 50 | 56 | 75 | 100 |
|---|---|---|---|---|---|---|---|---|---|
| $CO_2$ proportion (%) | 100 | 92.5 | 85 | 70 | 60 | 50 | 44 | 25 | 0 |
| $CF_3I$ required for extinction (vol.%) | 0 | 0.86 | 1.30 | 2.03 | 2.33 | 2.57 | 2.78 | 3.05 | 3.36 |
| $CO_2$ required for extinction (vol.%) | 20.74 | 10.63 | 7.34 | 4.73 | 3.49 | 2.57 | 2.18 | 1.02 | 0 |
| Sum of $CF_3I$ and $CO_2$ concentration (vol. %) | 20.74 | 11.49 | 8.64 | 6.76 | 5.82 | 5.13 | 4.96 | 4.06 | 3.36 |

Figure 2 shows the results of cup burner tests, in which the extinguishing concentration is plotted as a function of the proportion of $CF_3I$ in the blend. The fact that the data points are not in a straight line but show considerable curvature: looking at the third column, just 0.86% $CF_3I$ reduced the quantity of $CO_2$ required by almost half (20.74% → 10.63%). This is clear evidence of synergy. This plot can be used to determine optimal blends, particularly if other factors such as such as liquid density and vapour pressure are considered.

EXAMPLE 3 - PRESSURE-TEMPERATURE TESTS FOR $56CF_3I:44CO_2$ WITH DIFFERENT FILL DENSITIES

The 56 mol% $CF_3I$ 44 mol% $CO_2$ blend ("$56CF_3I:44CO_2$") was evaluated to determine whether it could fit into the same Halon 1301 container.

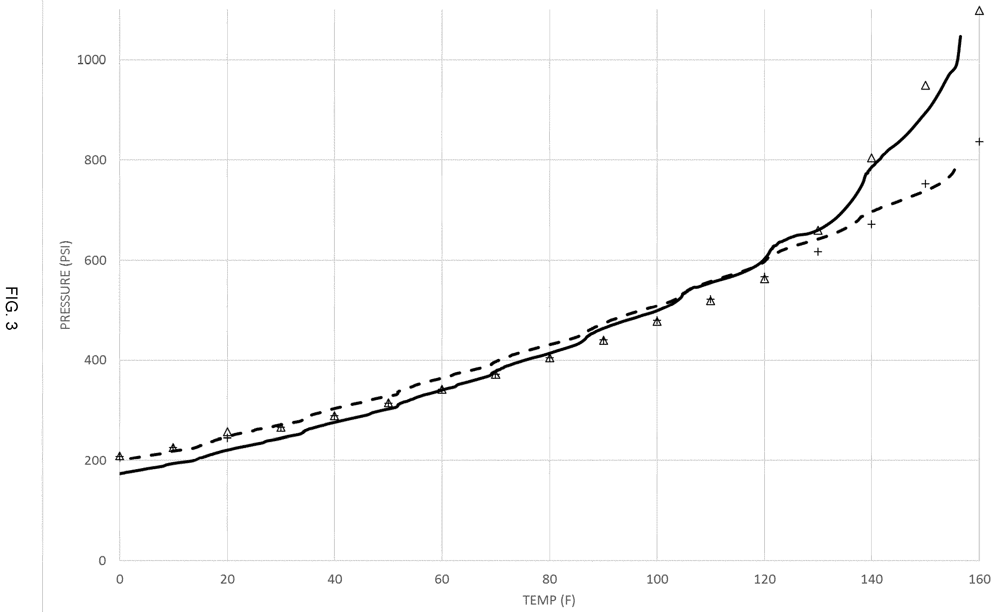

Pressure was monitored over a range of temperatures with certain fill densities of the $CF_3I/CO_2$ blend. The results are shown in Figure 3 which plots pressure over a range of temperatures, in which:

The dashed line represents $56CF_3I:44CO_2$ at 1.3x Halon fill density
The solid line represents $56CF_3I:44CO_2$ at 1.5x Halon fill density
Crosses represent a Halon 1301 curve at typical fill ratio
Triangles represent a Halon 1301 curve at max fill ratio

As shown in Figure 3, $56CF_3I:44CO_2$ blend with 1.3x and 1.5x fill density has very similar pressure-temperature behaviour to that of Halon 1301 HRD normal fill and max fill, respectively. In Figure 3, values expressed in psig are to be converted in KPa, with 1 psig=6.89476 kPa.

The above examples demonstrate that blends according to the present disclosure provide a "drop-in" replacement for Halon 1301, where relative mass, relative volume, and relative pressure are optimized.

References to "comprises" and/or "comprising," should be understood to also encompass "consist(s) of", "consisting of", "consist(s) essentially of" and "consisting essentially of".

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof.

While the present disclosure has been described with reference to an exemplary embodiment or embodiments, it will be understood by those skilled in the art that various changes may be made and equivalents may be substituted for elements thereof without departing from the scope of the present disclosure. In addition, many modifications may be made to adapt a particular situation or material to the teachings of the present disclosure without departing from the essential scope thereof. Therefore, it is intended that the present disclosure not be limited to the particular embodiment disclosed as the best mode contemplated for carrying out this present disclosure, but that the present disclosure will include all embodiments falling within the scope of the claims.

## Claims

1. A fire suppression composition comprising $CF_3I$ and $CO_2$,

   wherein said $CF_3I$ is present in an amount of from 53 mol.% to 59 mol.%, based on the total moles of $CF_3I$ and $CO_2$ present in the fire suppression composition; and
   wherein the total amount of additional components, if any, in said fire suppression composition is up to 18 weight%, based on the total weight of the fire suppression composition.

2. The fire suppression composition according to claim 1, wherein said $CF_3I$ is present in an amount from 54 mol.% to 58 mol.%, based on the total moles of $CF_3I$ and $CO_2$ present in the fire suppression composition.

3. The fire suppression composition according to claim 1, wherein said $CF_3I$ is present in an amount from 55 mol.% to 58 mol.%, based on the total moles of $CF_3I$ and $CO_2$ present in the fire suppression composition.

4. The fire suppression composition according to any preceding claim wherein said composition further comprises one or more additional components.

5. The fire suppression composition according to any preceding claim, wherein the additional components are selected from one or more gases, additional fire suppressant compounds, odorants, or combinations thereof.

6. The fire suppression composition according to any preceding claim, wherein the total amount of additional components is up to 15 weight%, based on the total weight of the fire suppression composition.

7. The fire suppression composition according to claim 6, wherein the total amount of additional components is up to 5 weight%, based on the total weight of the fire suppression composition.

8. A fire suppression system or device containing the fire suppression composition as claimed in any preceding claim.

9. The fire suppression system or device according to claim 8, wherein said fire suppression system additionally comprises a dispensing component.

10. A method for extinguishing a fire comprising using a fire suppression composition as claimed in any one of claims 1-7.

11. A method for preparing a fire suppression composition as claimed in any one of claims 1-7, said method comprising the steps of (i) providing $CF_3I$, (ii) providing $CO_2$ and (iii) combining $CF_3I$ and $CO_2$ to form a fire suppression composition as defined in any one of claims 1-7.

12. The method according to claim 11, wherein said method further comprises the additional step of adding one or more additional components.

## Patentansprüche

1. Feuerlöschzusammensetzung, umfassend $CF_3I$ und $CO_2$,

   wobei das $CF_3I$ in einer Menge von 53 Mol-% bis 59 Mol-% vorhanden ist, basierend auf den in der Feuerlöschzusammensetzung vorhandenen Gesamtmolen von $CF_3I$ und $CO_2$; und
   wobei die Gesamtmenge von zusätzlichen Komponenten, sofern vorhanden, in der Feuerlöschzusammensetzung bis zu 18 Gewichtsprozent beträgt, basierend auf dem Gesamtgewicht der Feuerlöschzusammensetzung.

2. Feuerlöschzusammensetzung nach Anspruch 1, wobei das $CF_3I$ in einer Menge von 54 Mol-% bis 58 Mol-% vorhanden ist, basierend auf den in der Feuerlöschzusammensetzung vorhandenen Gesamtmolen von $CF_3I$ und $CO_2$.

3. Feuerlöschzusammensetzung nach Anspruch 1, wobei das $CF_3I$ in einer Menge von 55 Mol-% bis 58 Mol-% vorhanden ist, basierend auf den in der Feuerlöschzusammensetzung vorhandenen Gesamtmolen von $CF_3I$ und

$CO_2$.

4. Feuerlöschzusammensetzung nach einem der vorhergehenden Ansprüche, wobei die Zusammensetzung ferner eine oder mehrere zusätzliche Komponenten umfasst.

5. Feuerlöschzusammensetzung nach einem der vorhergehenden Ansprüche, wobei die zusätzlichen Komponenten ausgewählt sind aus einem oder mehreren Gasen, zusätzlichen Feuerlöschverbindungen, Geruchsstoffen oder Kombinationen davon.

6. Feuerlöschzusammensetzung nach einem der vorhergehenden Ansprüche, wobei die Gesamtmenge von zusätzlichen Komponenten bis zu 15 Gewichtsprozent beträgt, basierend auf dem Gesamtgewicht der Feuerlöschzusammensetzung.

7. Feuerlöschzusammensetzung nach Anspruch 6, wobei die Gesamtmenge von zusätzlichen Komponenten bis zu 5 Gewichtsprozent beträgt, basierend auf dem Gesamtgewicht der Feuerlöschzusammensetzung.

8. Feuerlöschsystem oder -vorrichtung, das die Feuerlöschzusammensetzung nach einem der vorhergehenden Ansprüche enthält.

9. Feuerlöschsystem oder -vorrichtung nach Anspruch 8, wobei das Feuerlöschsystem zusätzlich eine Abgabekomponente umfasst.

10. Verfahren zum Löschen eines Feuers, umfassend Verwenden einer Feuerlöschzusammensetzung nach einem der Ansprüche 1 bis 7.

11. Verfahren zum Herstellen einer Feuerlöschzusammensetzung nach einem der Ansprüche 1 bis 7, wobei das Verfahren die Schritte (i) Bereitstellen von $CF_3I$, (ii) Bereitstellen von $CO_2$ und (iii) Kombinieren von $CF_3I$ und $CO_2$ unter Ausbilden einer Feuerlöschzusammensetzung nach einem der Ansprüche 1 bis 7 umfasst.

12. Verfahren nach Anspruch 11, wobei das Verfahren ferner den zusätzlichen Schritt Hinzufügen einer oder mehrerer zusätzlicher Komponenten umfasst.

**Revendications**

1. Composition de lutte contre les incendies comprenant du $CF_3I$ et du $CO_2$,

dans laquelle ledit $CF_3I$ est présent dans une quantité comprise entre 53 % en moles et 59 % en moles, sur la base du nombre total de moles de $CF_3I$ et de $CO_2$ présentes dans la composition de lutte contre les incendies ; et dans laquelle la quantité totale de composants supplémentaires, le cas échéant, dans ladite composition de lutte contre les incendies est jusqu'à 18 % en poids, sur la base du poids total de la composition de lutte contre les incendies.

2. Composition de lutte contre les incendies selon la revendication 1, dans laquelle ledit $CF_3I$ est présent dans une quantité comprise entre 54 % en moles et 58 % en moles, sur la base du nombre total de moles de $CF_3I$ et de $CO_2$ présentes dans la composition de lutte contre les incendies.

3. Composition de lutte contre les incendies selon la revendication 1, dans laquelle ledit $CF_3I$ est présent dans une quantité comprise entre 55 % en moles et 58 % en moles, sur la base du nombre total de moles de $CF_3I$ et de $CO_2$ présentes dans la composition de lutte contre les incendies.

4. Composition de lutte contre les incendies selon une quelconque revendication précédente, dans laquelle ladite composition comprend également un ou plusieurs composants supplémentaires.

5. Composition de lutte contre les incendies selon une quelconque revendication précédente, dans laquelle les composants supplémentaires sont choisis dans un ou plusieurs gaz, des composés de lutte contre les incendies supplémentaires, des odorants ou des combinaisons de ceux-ci.

**6.** Composition de lutte contre les incendies selon une quelconque revendication précédente, dans laquelle la quantité totale de composants supplémentaires est jusqu'à 15 % en poids, sur la base du poids total de la composition de lutte contre les incendies.

**7.** Composition de lutte contre les incendies selon la revendication 6, dans laquelle la quantité totale de composants supplémentaires est jusqu'à 5 % en poids, sur la base du poids total de la composition de lutte contre les incendies.

**8.** Système ou dispositif de lutte contre les incendies contenant la composition de lutte contre les incendies selon une quelconque revendication précédente.

**9.** Système ou dispositif de lutte contre les incendies selon la revendication 8, dans lequel ledit système de lutte contre les incendies comprend en outre un composant de distribution.

**10.** Procédé d'extinction d'un incendie comprenant l'utilisation d'une composition de lutte contre les incendies selon l'une quelconque des revendications 1 à 7.

**11.** Procédé de préparation d'une composition de lutte contre les incendies selon l'une quelconque des revendications 1 à 7, ledit procédé comprenant les étapes consistant à (i) fournir du $CF_3I$, (ii) fournir du $CO_2$ et (iii) combiner du $CF_3I$ et du $CO_2$ pour former une composition de lutte contre les incendies selon l'une quelconque des revendications 1 à 7.

**12.** Procédé selon la revendication 11, dans lequel ledit procédé comprend également l'étape supplémentaire d'ajout d'un ou de plusieurs composants supplémentaires.

FIG. 1

FIG. 2

FIG. 3

**EP 3 985 086 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150041157 A
- KR 20010038267
- CN 106281232
- WO 2019099961 A